# EUROPEAN PATENT APPLICATION

(11) **EP 3 466 332 A1**
(43) Date of publication of application: **10.04.2019**
(21) Application number: 17802952.6
(22) Date of filing: 26.05.2017
(51) Int. Cl.: A61B 5/05, A61N 1/05, A61N 1/20

(54) **PROBE, EPIDERMAL ELECTRIC POTENTIAL MEASURING DEVICE, EPIDERMAL ELECTRIC POTENTIAL MEASURING METHOD, AND COSMETIC METHOD**

(30) Priority: 27.05.2016 JP 2016106831
(71) Applicant: Tohoku University, Sendai-shi, Miyagi 980-8577 (JP)
(72) Inventor: NISHIZAWA, Matsuhiko, Sendai-shi Miyagi 980-8577 (JP); NAGAMINE, Kuniaki, Sendai-shi Miyagi 980-8577 (JP); ABE, Yuina, Sendai-shi Miyagi 980-8577 (JP); YAMASAKI, Kenshi, Sendai-shi Miyagi 980-8577 (JP)
(74) Representative: Scott, Stephen John
(86) International application number: PCT/JP2017/020529
(87) International publication number: WO 2017/204366

(57) **Abstract**

Disclosed are a probe including a hollow needle having an outer diameter of 0.003 to 0.4 mm and a length of 0.2 to 40 mm, wherein a distal end and a terminal end of the hollow needle are bridged with a salt bridge, an epidermal electric potential measuring device including the probe, an epidermal electric potential measuring method using the epidermal electric potential measuring device, and a cosmetic method using the epidermal electric potential measuring device.

## Description

### TECHNICAL FIELD

This disclosure relates to a probe, an epidermal electric potential measuring device, an epidermal electric potential measuring method, and a cosmetic method.

### BACKGROUND

The stratum corneum layer of the outermost layer of the skin has a barrier function to prevent the invasion of external foreign matters and evaporation of moisture from the body. For example, in atopic dermatitis, which is one of the skin diseases, the barrier dysfunction causes an inflammatory reaction and significantly decreases the patient's quality of life (QOL). In order to develop a method for the prevention and the treatment of these diseases, it is required to reveal the onset mechanism at the tissue and the cell levels. However, it is difficult to analyze and determine the changes of the barrier function inside the skin only from its appearance, and it is necessary to develop a system that can quantify the changes.

An epidermal electric potential is an electric potential difference generated along the thickness direction of the epidermal layer immediately beneath the stratum corneum and varies due to the dysfunction of the barrier. In this regard, an electrotherapeutic method, in which an electric potential is applied to the skin to promote the cure of the disrupted barrier function, has been reported (see Patent Literature 1). It is considered that the effect of recovering the barrier function is based on controlling the epidermal electric potential, but the detailed mechanism is unknown. According to the method described in Patent Literature 1, the electrical stimulation is applied between the electrodes attached on the surface of the body, so that it is difficult to affect the epidermal electric potential directly.

### CITATION LIST

### Patent Literature

PTL 1 : JP 2002-291909 A
PTL 2 : JP 2002-177228 A

### Non-Patent Literature

NPL 1 ; K.S.Turner, D.O.N.W.Powell, C.N.Carney and R.O.Y.C.Orlando, 286 (1978)

### SUMMARY

### (Technical Problem)

In order to measure the epidermal electric potential, it is necessary to locate an electrode both on the surface and in the inside (in the dermal layer right under the epidermis) of the body.

In the present situation, an invasive technique, which requires hurting the skin to expose the dermal layer for placing the electrode, has been utilized for animals at the research level, but the application of such method to a person is accompanied with danger. As for a non-invasive technique, there is a method in which a salt bridge bridged to the electrode is put under the tongue instead of under the epidermis, but it is poor in quantitativeness because of the measurement of the potential at the site far from the sublingual region results (see PTL 2).

As related studies, some experiments have been conducted to measure a transepithelial electric potential generated in the mucosal tissues of internal organs such as the trachea and the gastrointestinal tract (see NPL 1). In these studies, when the electrode is located right under the epithelium, a hypodermic injection needle is inserted under the epidermis of the forearm to be used as a reference of the electric potential. However, about 19 G (about 1.1 mm in diameter) needle, which is considerably thick among the injection needles available for human beings, is employed. In order to apply this measuring system to the human skin and measure the electric potential for a long time, the system is desirable to be less invasive to the human, such as reducing damage to the skin near the measuring site caused by insertion of the needle.

As described above, the conventional epidermal electric potential measuring methods largely remain to be improved in the points such as invasiveness, quantitativeness, and long-term usability.

Therefore, this disclosure aims to provide a probe, an epidermal electric potential measuring device, an epidermal electric potential measuring method, and a cosmetic method, which enable minimally invasive epidermal electric potential measurement by inserting a painless needle at an appropriate depth.

### (Solution to Problem)

The summary of this disclosure is as follows.

A probe of the disclosure comprises a hollow needle having an outer diameter of 0.003 to 0.4 mm and a length of 0.2 to 40 mm, and the distal end and the terminal end of the hollow needle are bridged with a salt bridge.

For the probe of the disclosure, it is preferable that the contact angle of water to the outer surface of the hollow needle is 1 to 175 °.

Further, for the probe of the disclosure, it is preferable that the outer surface of the hollow needle is treated with ultraviolet ozone.

Further, for the probe of the disclosure, it is preferable that the salt bridge comprises a hydrogel.

Further, for the probe of the disclosure, it is preferable that the hydrogel comprises a hydrogel material and a physiological salt solution.

An epidermal electric potential measuring device of the disclosure comprises the probe described above, a first electrode bridged to the terminal end of the hollow needle with the salt bridge, a second electrode connected to the first electrode by an electron conductive material, and another probe bridged to the second electrode with the salt bridge.

For the epidermal electric potential measuring device of the disclosure, it is preferable that the distance between the distal end of the probe and the distal end of the other probe in the direction perpendicular to the axial direction of the hollow needle is 1 to 50 mm.

Further, for the epidermal electric potential measuring device of the disclosure, it is preferable that a spacer is provided between the distal end and the terminal end of the hollow needle.

An epidermal electric potential measuring method of the disclosure employs the epidermal electric potential measuring device described above and comprises a probe disposition which includes disposing the probe to make its distal end reach the dermis and disposing the another probe to make its distal end positioned on the skin surface, and a voltage/current measuring process of measuring a voltage by applying a current or measuring a current by applying a voltage between the first electrode and the second electrode.

A cosmetic method of the disclosure employs the epidermal electric potential measuring device described above and comprises performing probe disposition which includes disposing the probe to make its distal end reach the dermis and disposing the other probe to make its distal end positioned on the skin surface, and performing voltage/current application which includes applying a voltage or a current between the first electrode and the second electrode.

### (Advantageous Effect)

According to this disclosure, the minimally invasive epidermal electric potential measuring method and the cosmetic method can be provided by inserting a painless needle at an appropriate depth.

### BRIEF DESCRIPTION OF THE DRAWINGS

In the accompanying drawings:

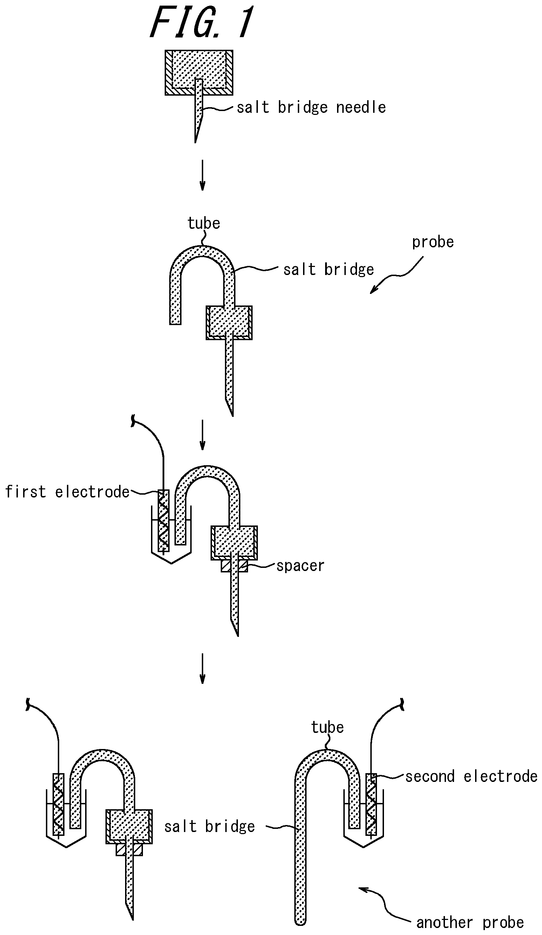

FIG. 1 illustrates one example of probes according to some of the embodiments of the disclosure, one example of epidermal electric potential measuring device according to some of the embodiments of the disclosure, and a manufacturing method thereof in the cross-sectional views.

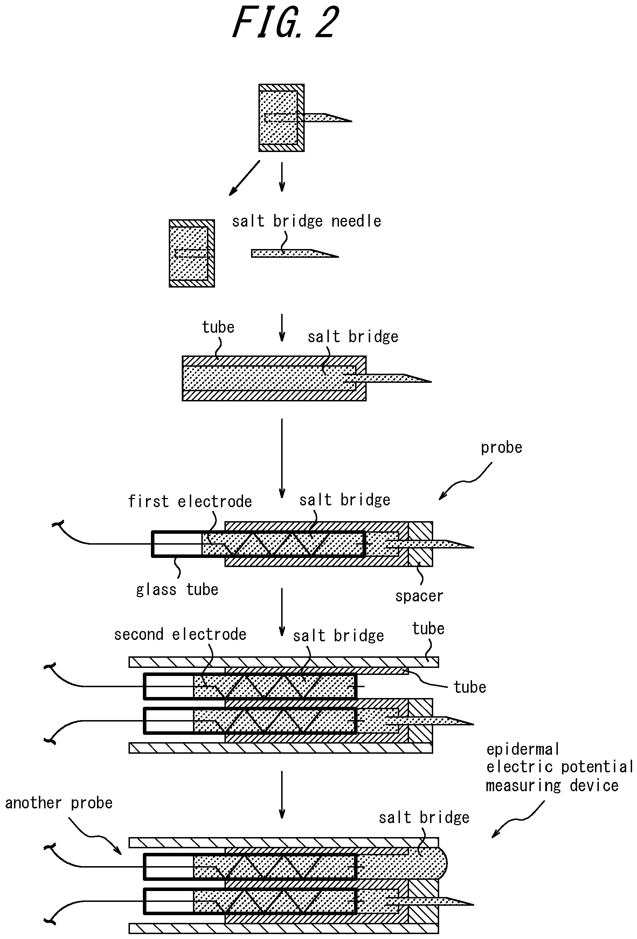

FIG. 2 illustrates another example of probes according to some of the embodiments of the disclosure, another example of epidermal electric potential measuring device according to some of the embodiments of the disclosure, and a manufacturing method thereof in the cross-sectional views.

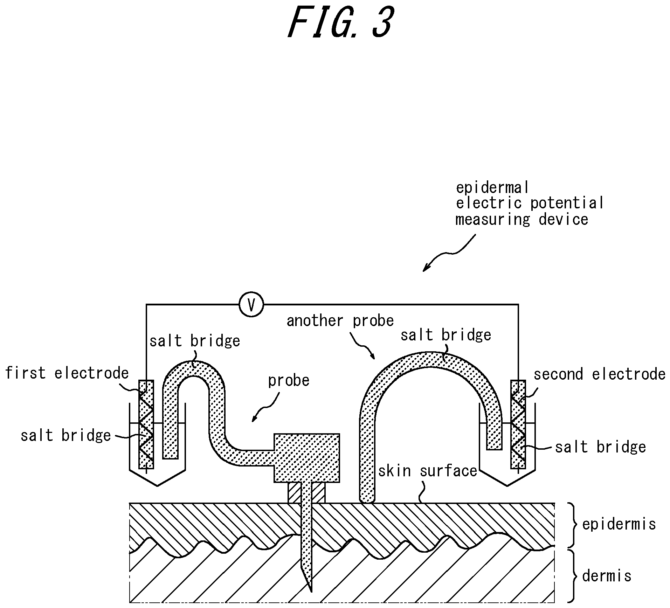

FIG. 3 illustrates one example of epidermal electric potential measuring method according to some of the embodiments of the disclosure, which employs one example of probes and one example of epidermal electric potential measuring device in FIG. 1.

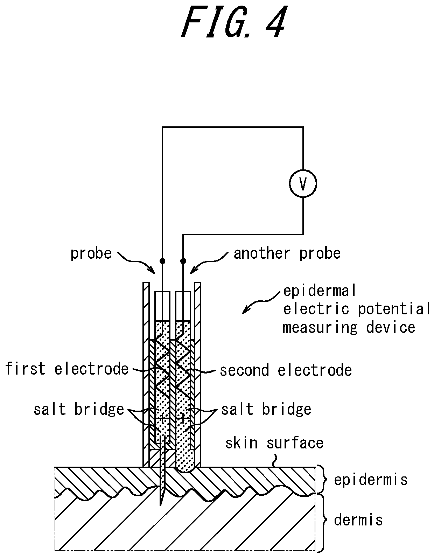

FIG. 4 illustrates another example of epidermal electric potential measuring method according to some of the embodiments of the disclosure, which employs another example of probes and another example of epidermal electric potential measuring device in FIG. 2.

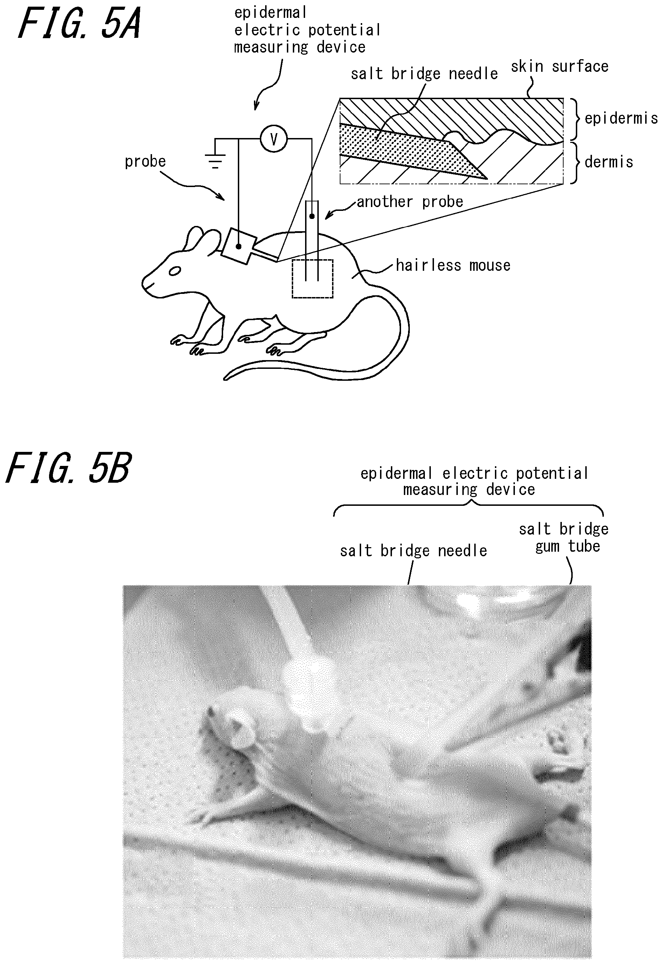

FIGS. 5A and 5B illustrate an outline of the experiment when the epidermal electric potential of a hairless mouse is measured by one example of epidermal electric potential measuring method according to some of the embodiments of the disclosure. FIGS. 5A is a diagram of the principle of the experiment, and FIGS. 5B is a photograph of the appearance during the experiment.

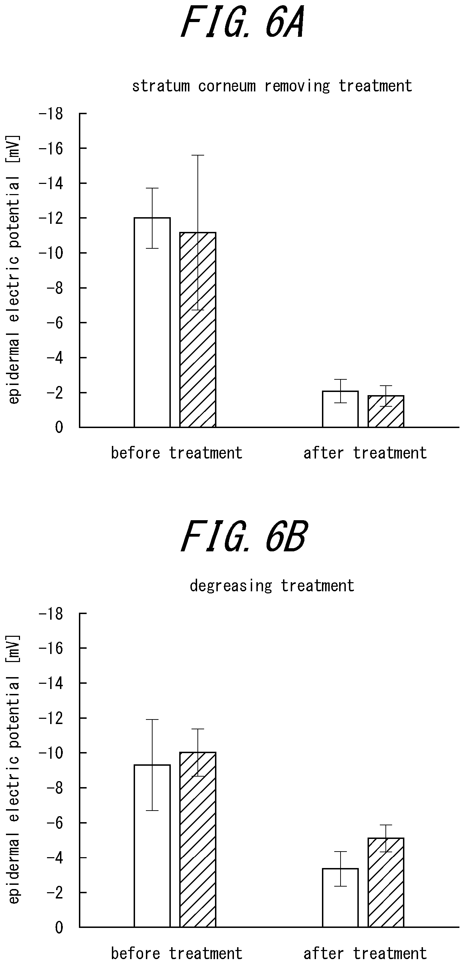

FIGS. 6A and 6B illustrate the results of measuring the epidermal electric potentials of the hairless mouse by one example of epidermal electric potential measuring method according to some of the embodiments of the disclosure. FIGS. 6A is a graph of the results when stratum corneum removing treatment was given, and FIGS. 6B is a graph of the results when degreasing treatment was given. In FIGS. 6A and FIGS. 6B, vertical lines indicate error bars (mean ± standard deviation) of three measurements.

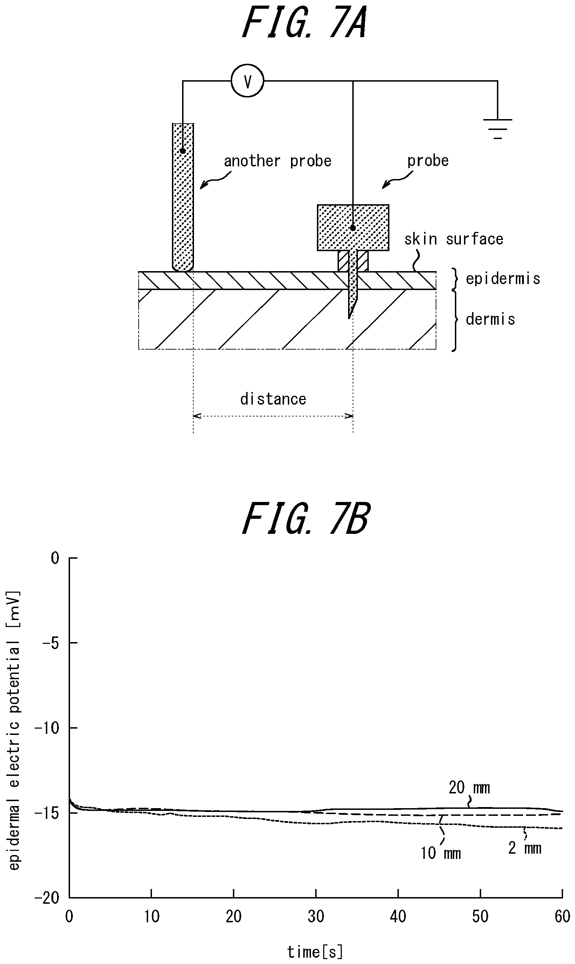

FIGS. 7A and 7B illustrate an outline and results of investigating the preferable distance between the distal end of the probe according to some of the embodiments of the disclosure and the distal end of the other probe according to some of the embodiments of the disclosure by using porcine skin. FIG. 7A is a diagram for the outline of the experiment, and FIG. 7B is a graph presenting the results of the experiment.

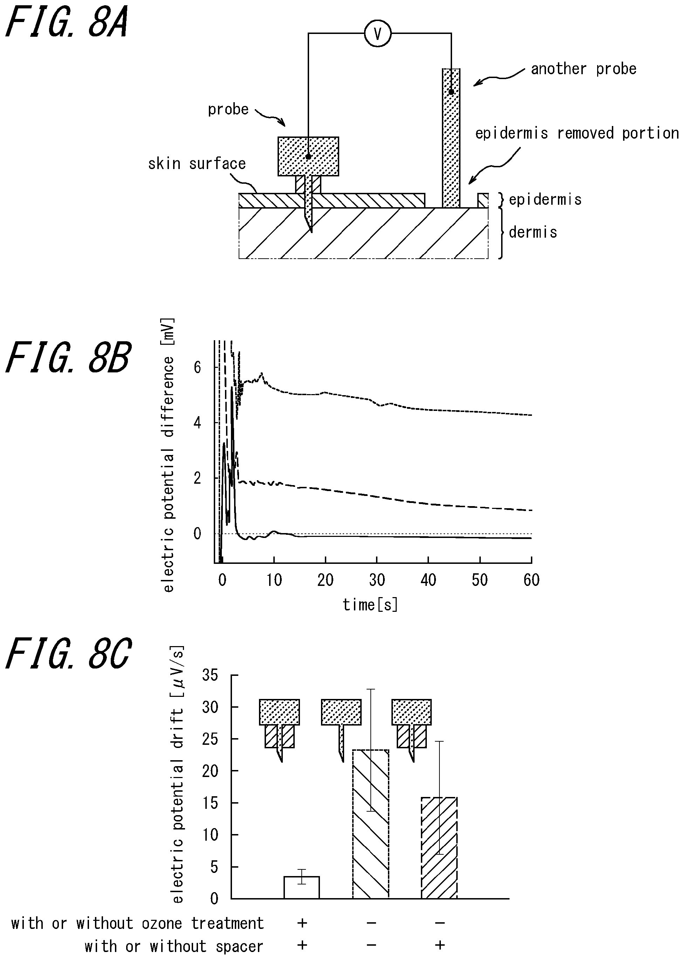

(FIGS. 8A, 8B, and 8C) FIGS. 8A, 8B, and 8C illustrate an outline and results of investigating the preferable length of the hollow needle in the probe according to some of the embodiments of the disclosure and the preferable treatment for the outer surface of the hollow needle by using porcine skin subjected to epidermis removing treatment. FIG. 8A is a diagram for the outline of the experiment, and FIGS. 8B and 8C are graphs presenting the results of the experiment. In FIG. 8C, vertical lines indicate error bars (mean±standard deviation) of three measurements.

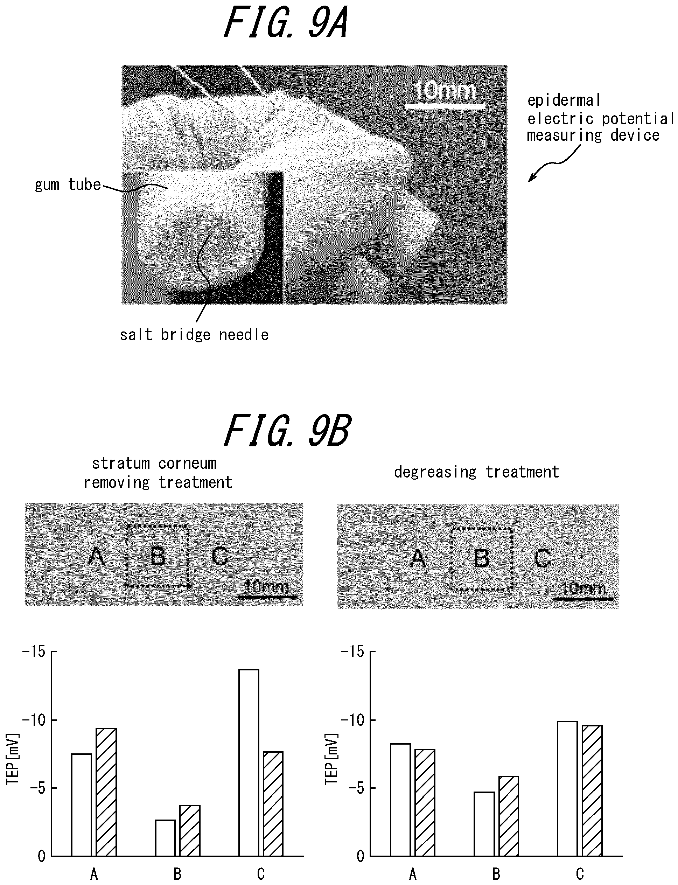

FIGS. 9A and 9B illustrate an outline and results of measuring the epidermal electric potentials of porcine skins by another example of epidermal electric potential measuring method according to some of the embodiments of the present disclosure. FIG. 9A is a photograph of the epidermal potential measuring device, FIG. 9B presents a photograph and a graph for the outline and the results of the experiment, respectively, when stratum corneum removing treatment was given, and another photograph and another graph for the outline and the results of the experiment, respectively, when degreasing treatment was given. In FIGS. 9B, A and C represent the untreated sites, and B represents the treated site.

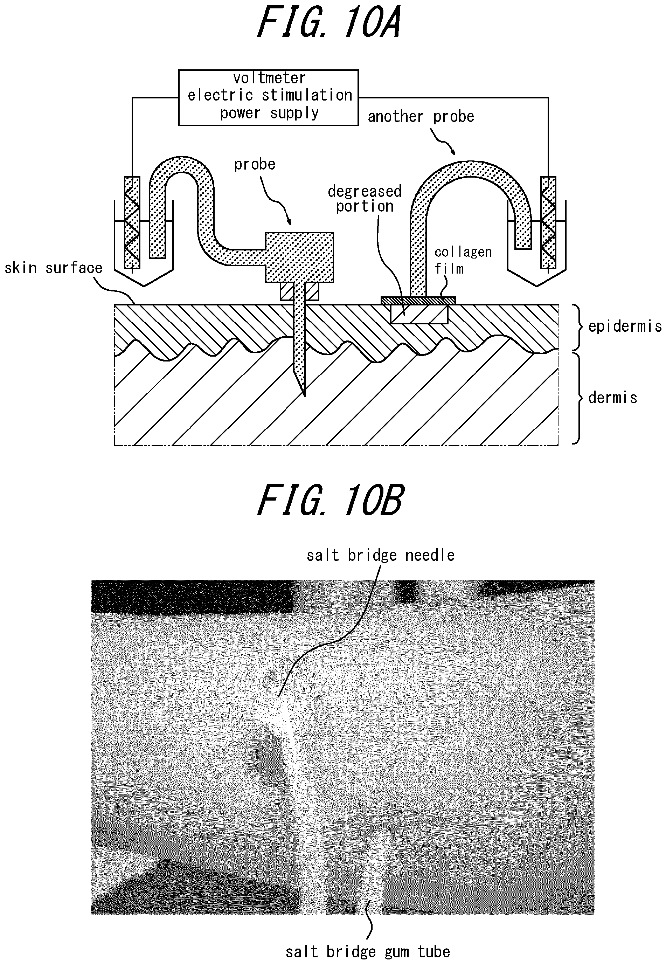

FIGS. 10A and 10B illustrate an outline of measuring the epidermal electric potential on the human forearm by another example of epidermal electric potential measuring method according to some of the embodiments of the present disclosure. FIG. 10A is a diagram for the outline of the experiment, and FIG. 10B is a photograph of the appearance during the experiment.

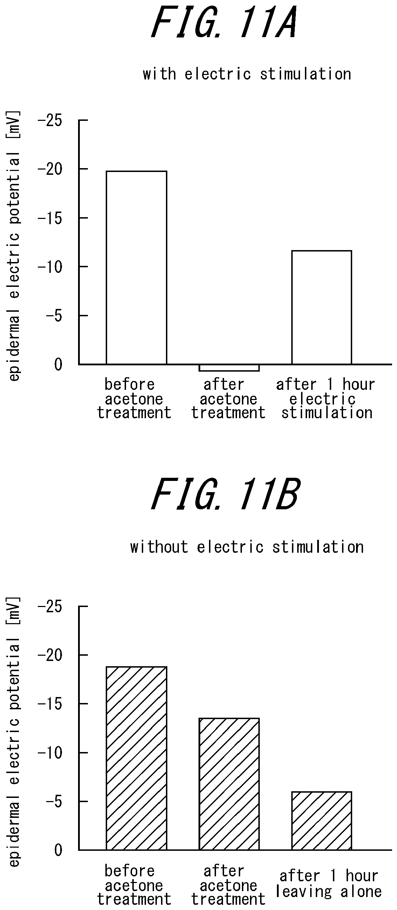

FIGS. 11A and 11B illustrate results of measuring the epidermal electric potential on the human forearm by another example of epidermal electric potential measuring method according to some of the embodiments of the present disclosure. FIG. 11A is a graph for the results when a voltage is applied for 1 hour after degreasing treatment, and FIG. 11B is a graph for the results when no voltage is applied to be left alone for 1 hour after degreasing treatment.

### DETAILED DESCRIPTION

Some embodiments of the probe for the epidermal electric potential measurement, the epidermal electric potential measuring device, the epidermal electric potential measuring method, and the cosmetic method of the disclosure will be exemplified and described in detail below with reference to the figures.

### (Probe)

A probe for the epidermal electric potential measurement in some of the embodiments of the disclosure (hereinafter also referred to as "some of the embodiments") comprises a hollow needle having an outer diameter of 0.003 to 0.4 mm and a length of 0.2 to 40 mm.

The probe for the epidermal electric potential measurement in some of the embodiments is the probe wherein the distal end and the terminal end of the hollow needle are bridged with a salt bridge.

The probe for the epidermal electric potential measurement in some of the embodiments is used by being disposed to make the distal end of the hollow needle reach the dermis and is preferably used to measure the electric potential between the dermis and the skin surface, in other words, the epidermal electric potential (described later).

Since the distal end of the hollow needle is bridged to its terminal end with the salt bridge, ionic conductivity is secured between the distal end and the terminal end, and the electric potential near the distal end of the hollow needle can be measured as the electric potential near the terminal end of the hollow needle (if needed beyond the terminal end of the hollow needle).

The conventional epidermal electric potential measurement requires a highly invasive operation such as insertion of the electrode into the dermis, whereas the epidermal electric potential measurement in some of the embodiments can provide a minimally invasive epidermal electric potential measurement.

FIG. 1 illustrates one example of probes according to some of the embodiments of the disclosure, one example of epidermal electric potential measuring device according to some of the embodiments of the disclosure, and a manufacturing method thereof in the cross-sectional views.

FIG. 2 illustrates another example of probes according to some of the embodiments of the disclosure, another example of epidermal electric potential measuring device according to some of the embodiments of the disclosure, and a manufacturing method thereof in the cross-sectional views.

One example of probes illustrated in FIG. 1 and another example of probes illustrated in FIG. 2 comprise the same configuration in principal other than that the distal end of the probe and the first electrode are directly bridged in one example whereas both the distal end of the probe and the first electrode are immersed in an electrolytic solution (see FIG. 1) so as to be bridged with this electrolytic solution in another example.

From the point of reducing the invasiveness, for the probe for the epidermal electric potential measurement in some of the embodiments, the followings are preferable:
The outer diameter of the hollow needle is 0.003 mm or more, preferably 0.005 mm or more, further preferably 0.01 mm or more, more preferably 0.02 mm or more, even more preferably 0.05 mm or more, and preferably 0.4 mm or less, further preferably 0.35 mm or less, more preferably 0.3 mm or less, even more preferably 0.25 mm or less.

The inner diameter of the hollow needle is preferably 0.001 mm or more, further preferably 0.002 mm or more, more preferably 0.005 mm or more, even more preferably 0.01 mm or more, and preferably 0.3 mm or less, further preferably 0.25 mm or less, more preferably 0.2 mm or less, even more preferably 0.15 mm or less.

Wherein, the outer diameter and the inner diameter refer to each the maximum diameter at the distal end.

The size of the hollow needle can be 27 G, 28 G, 29 G, 30 G, 31 G, 32 G, 33 G, and 34 G according to the standard.

The length of the hollow needle is 0.2 to 40 mm.

Wherein, the length of the hollow needle refers to the length from the distal end to the terminal end of the hollow needle, more specifically, the length of the part that can be inserted.

In general, since human skin consists of epidermis (thickness: 200 µm), dermis (thickness 1 to 3 mm) and subcutaneous tissue in order from the outer surface side, 0.2 mm or more length of the hollow needle would make it possible for the distal end of the hollow needle to reach the dermis through the epidermis in using the probe. In case that the part of the predetermined length can not be inserted against the elasticity of the skin, the length of the hollow needle is preferably 0.25 mm or more, further preferably 0.3 mm or more, more preferably 0.5 mm or more, even more preferably 0.7 mm or more, and particularly preferably 1 mm or more.

In addition, as shorter salt bridge in length is also required in order to improve the precision of the epidermal electric potential measurement, 40 mm or less length of the hollow needle would make it possible to measure the electric potential near the distal end of the hollow needle as the electric potential near the terminal end of the needle without loss of the electric potential. Wherein, the length of the hollow needle is preferably 38 mm or less, further preferably 20 mm or less, more preferably 10 mm or less, even more preferably 4 mm or less, particularly preferably 2 mm or less.

Regarding the shape of the hollow needle, the shape of the outline of the outer surface in the cross section along the axial direction, particularly at the distal end of the hollow needle, can be tapered, with gradually decreasing the width of the hollow space of the hollow needle in the cross-section from the terminal end to the distal end, on one side or both sides along the direction perpendicular to the axial direction.

Regarding the shape of the hollow needle, the shape of the outline of the outer surface in the cross-section perpendicular to the axial direction can be circle (circle, ellipse etc.) or polygon (square, rectangle, triangle, etc.), wherein the corner of the rectangle can be rounded and preferably can be circle from the viewpoint of reducing invasiveness.

The material for the hollow needle can include, for example, but not limited to, a resin, an oxide, and a metal.

The resin can include silicone, polycarbonate, acrylonitrile-butadiene-styrene (ABS) resin, and phenol resin.

The oxide can include inorganic oxides and derivatives thereof. Wherein, the inorganic oxides can include silicon oxide, tin oxide, zirconia oxide, titanium oxide, niobium oxide, tantalum oxide, aluminum oxide, tungsten oxide, hafnium oxide, zinc oxide.

The metal can include nickel, iron, stainless steel, cobalt-chromium alloy, titanium, titanium alloy and silicon (Si), and is, in particular from the points of mechanical strength and biological safety, preferably metallic biomaterial such as stainless steel, cobalt chromium alloy, titanium and titanium alloy.

In some of the embodiments, the contact angle of water to the outer surface of the hollow needle is not particularly limited, but can be 1 to 175 °°, to quickly make the contact between the hollow needle and the interstitial fluid immediately after the insertion, preferably 150 ° or less, further preferably 120 ° or less, more preferably 90 ° or less, particularly preferably 60 ° or less, and most preferably 30 ° or less, and preferably 1.5 ° or more, further preferably 2 ° or more, more preferably 3 ° or more, and particularly preferably 4 ° or more.

Wherein the contact angle of water to the outer surface of the hollow needle refers to an average of contact angles measured according to JIS R3257 at any points on the outer surface of the hollow needle.

In addition, in some of the embodiments, it is preferable that the contact angle of water to the inner surface of the hollow needle also has the same range as that of the contact angle of water to the outer surface of the hollow needle.

In some of the embodiments, the surface of the hollow needle can be treated with a surface treatment in order to make the contact angle of water to the surface of the hollow needle within the above range.

Such surface treatment includes ultraviolet ozone treatment, oxygen plasma treatment, treatment with a silane coupling agent, etching treatment using chemicals such as acid and alkali, corona treatment, atmospheric plasma treatment, and flame treatment, and is preferably ozone treatment, oxygen plasma treatment, corona treatment, and atmospheric plasma treatment from the point of safe treatment without using chemicals that can influence living bodies.

In some of the embodiments, as illustrated in FIGS. 1 and 2, a spacer can be provided between the distal end and the terminal end of the hollow needle. In particular, in these exemplary probes, a ring-shaped spacer is provided so that its central hole is fitted into by the hollow needle.

In some of the embodiments, when the spacer is provided, the length of the hollow needle refers to the length from the distal end of the hollow needle to the spacer.

By providing the spacer, particularly by adjusting the axial length of the hollow needle, it is possible to adjust the length of the hollow needle appropriately depending on the purpose and the application. For example, when the length of the hollow needle is 4 mm, the length of the spacer is set to 3 mm so that 1 mm of the distal end of the hollow needle protrudes to reach the dermis.

The length in the axial direction of the spacer can be determined appropriately according to the desired length of the hollow needle.

The material for the spacer includes a resin, an oxide, and a metal, and is preferably the resin from the point of processability.

The salt bridge in some of the embodiments preferably comprises a hydrogel, an ionogel (a gel containing an ionic liquid), an electrolyte solution and an ionic liquid. The salt bridge can comprise only one of these or a combination of two or more of these.

It is preferable that the hydrogel is used to be filled into tubular material from the point of easily manufacturing.

It is preferable that the hydrogel comprises a hydrogel material and a physiological salt solution.

The hydrogel material is a material that forms the hydrogel by being dispersed in water (dispersion medium).

The hydrogen material contains natural polymer including agar, gelatin, agarose, xanthan gum, gellan gum, sclerotium gum, arabiya gum, tragacanth gum, karaya gum, cellulose gum, tamarind gum, guar gum, locust bean gum, glucomannan, chitosan, carrageenan, quince seed, galactan, mannan, starch, dextrin, curdlan, casein, pectin, collagen, fibrin, peptide, chondroitin sulfate such as sodium chondroitin sulfate, hyaluronic acid (mucopolysaccharide) and hyaluronic acid salt such as sodium hyaluronate, alginic acid, alginate such as sodium alginate and calcium alginate, and derivatives thereof; cellulose derivatives including methylcellulose, hydroxymethylcellulose, hydroxyethylcellulose, hydroxypropylcellulose, hydroxypropylmethylcellulose, carboxymethylcellulose and salts thereof; poly (meth) acrylic acids including polyacrylic acid, polymethacrylic acid, acrylic acid/alkyl methacrylate copolymer and salts thereof; synthetic polymer including polyvinyl alcohol, polyhydroxyethyl methacrylate, polyacrylamide, poly (N-isopropyl acrylamide), polyvinyl pyrrolidone, polystyrene sulfonic acid, polyethylene glycol, carboxyvinyl polymer, alkyl modified carboxyvinyl polymer, maleic anhydride copolymer, polyalkylene oxide type resin, crosslinked product of poly (methyl vinyl ether-alt-maleic anhydride) and polyethylene glycol, polyethylene glycol crosslinked product, N-vinylacetamide crosslinked product, acrylamide crosslinked product, starch·acrylate graft copolymer crosslinked product; silicone; interpenetrating network hydrogel and semi-interpenetrating network hydrogel; and mixtures of two or more of these.

Among them, it is preferable that the hydrogel material forming the hydrogel is agar, agarose, and gelatin from the point of high ionic conductivity, electrical neutrality, biological safety, and structural stability.

The physiological salt solution includes a saline, a buffered saline, and Ringer's solution, and is preferably Ringer's solution from the point of biocompatibility.

The saline preferably has a salt concentration of 0.85 to 0.95 wt%.

The buffered saline can be a saline in which a commercially available buffer is added, and includes, for example, phosphate buffered saline, Tris buffered saline, and HEPES buffered saline.

The Ringer's solution preferably has a composition of 0.53 to 0.58 wt% of chlorine ions and 0.03 to 0.036 wt% of calcium chloride dihydrate. As commercially available products of Ringer's solution, Solulact S, Lactec, Hartmann, Potacol R, Sol Acetate F, Vienna F, Physio, and Vicarbon are suitably used.

The methods for manufacturing the probe for the epidermal electric potential measurement in some of the embodiments can include, but not limited to, methods employing some of the usual skills in the art, and specifically, the methods described in Examples (see FIG. 1 and FIG. 2).

### (Epidermal electric potential measuring device)

The epidermal electric potential measuring device in some of the embodiments comprises one probe in some of the embodiments, a first electrode bridged to the distal end of the hollow needle via a salt bridge, a second electrode connected to the first electrode via electron conductive material and another probe bridged to the second electrode via a salt bridge.

The epidermal electric potential measuring device in some of the embodiments can be usually connected with a device having the function of a voltmeter between the first electrode and the second electrode via electron conductive material (see FIG. 3 and FIG. 4).

As described above, the epidermal electric potential measuring device in some of the embodiments is used as the probe in some of the embodiments is disposed to make the distal end of the hollow needle reach the dermis, and the electric potential between the dermis and the skin surface, the epidermal electric potential in other words, is measured.

Since the distal end and the terminal end of the hollow needle, and the terminal end of the hollow needle and the first electrode are bridged each other with the salt bridge, and since the distal end and the terminal end of the other probe, and the terminal end of the other probe and the second electrode are bridged each other with the salt bridge, it is possible to measure the electric potential difference of the epidermal surface at the distal end of the other probe to the dermis near the distal end of the hollow needle.

As described above, although the conventional epidermal electric potential measurement requires the highly invasive operation such as inserting the electrode into the dermis, the epidermal electric potential measurement in some of the embodiments can provide the minimally invasive epidermal electric potential measurement.

In some of the embodiments, the distance between the distal end of the probe and the distal end of the other probe in the direction perpendicular to the axial direction of the hollow needle is preferably 1 mm or more, more preferably 2 mm or more and further preferably 3 mm or more, and preferably 50 mm or less, more preferably 30 mm or less and further preferably 20 mm or less from the point of minimizing influences by the electric potential generated in the body (see FIGS. 1, 2, 7A, 7B).

Moreover, in some of the embodiments, the distance between the distal end of the probe and the distal end of the other probe in the axial direction of the hollow needle can be long enough to contain the length of the hollow needle from the viewpoint of disposing the electrodes each other across the epidermis (see FIGS. 1, 2, 7A, 7B).

The above-described distance in the direction perpendicular to the axial direction of the hollow needle and in the axial direction of the hollow needle can be appropriately adjusted by integrating the part including the first electrode and the part including the second electrode like another example of the epidermal electric potential measuring device illustrated in FIG. 2.

### (Epidermal electric potential measuring method)

The epidermal electric potential measuring method in some of the embodiments uses the epidermal electric potential measuring device in some of the embodiments and comprises performing probe disposition which includes disposing the probe of some of the disclosed embodiments to make its distal end reach the dermis and disposing the other probe different from the probe in some of the embodiments to make its distal end positioned on the skin surface, and voltage/current measurement which includes measuring a voltage by applying a current or measuring a current by applying a voltage between the first electrode and the second electrode.

The probe in some of the embodiments can be a probe for a reference electrode, and the other probe different from the probe in some of the embodiments can be a probe for the epidermal electric potential measurement.

FIG. 3 illustrates one example of epidermal electric potential measuring method according to some of the embodiments of the disclosure, which employs one example of probes and one example of epidermal electric potential measuring device in FIG. 1.

FIG. 4 illustrates another example of epidermal electric potential measuring method according to some of the embodiments of the disclosure, which employs another example of probes and another example of epidermal electric potential measuring device in FIG. 2.

One exemplary method illustrated in FIG. 3 and another exemplary method illustrated in FIG. 4 principally have the same configuration, other than the difference, in addition to the above-described differences between the probes of one example and the probes of another example, that the distance between the distal end of the probe and the distal end of the other probe (Described later) can be appropriately adjusted in one example, whereas the distance is fixed to the minimum in another example.

### ((Probe Disposition))

For disposing the probe in the probe disposition, the probe in some of the embodiments described above can be affixed on the skin surface, and the distal end of the hollow needle can reach the dermis by inserting the hollow needle of the probe through the skin surface to in vivo direction. Such method does not preferably correspond to a surgical procedure including puncture and also does not preferably correspond to a medical practice.

Moreover, it is not preferable that the distal end of the hollow needle reaches the subcutaneous tissue beneath the inner side of the dermis.

At this time, the hollow needle is preferably inserted in just one-time sting, that is without repeat of putting in and out the hollow needle, to achieve higher precision in the epidermal electric potential measurement.

In addition, the perpendicular insertion of the hollow needle to the skin surface would be preferable to make its distal end reach to the designed depth and to avoid the breakage and the damage caused by the penetration of the epidermis of the skin.

Moreover, in this process, it is adjustable for the distal end of the probe to reach the dermis by varying the length of the hollow needle of the epidermal electric potential measuring device in some of the disclosed embodiments.

For disposing the other probe in the probe disposition, the distal end of the other probe can be pressed against the skin surface.

### ((Voltage/Current Measurement))

The current value of the current applied and the voltage value of the voltage applied in the voltage/current measurement can be set appropriately according to the conditions of the experiment, but, from the point of obtaining a healthy skin with the functional barrier, it is preferable that the electric potential of the skin surface is negative (minus) to the electric potential in the dermis of the reference.

In some of the embodiments, in the voltage/current measurement, the operation can be varied depending on the magnitude relation of the voltage measured at a given time to a given standard voltage or the magnitude relation of the current measured at a given time to a given standard current.

From other points, the inventors found out a phenomenon that the barrier function of the skin was improved by applying, for example, a voltage of -0.5 V to the skin whose barrier function was deteriorated.

In some of the embodiments, considering this finding, the preferable embodim2ents include also the following.

In measuring the voltage and applying the voltage in the voltage/current measurement, when the measured voltage is greater than a given standard voltage at a given time-point, after the given time-point, it is preferable to apply the voltage and to measure the voltage further until the measured voltage becomes equal to or less than the given standard voltage.

For example, in the case of -10 mV of the standard voltage, when the measured voltage is -5 mV at a given time-point, after the given time-point, it is allowed to apply the voltage and to measure the voltage until the measured voltage becomes -15 mV.

In measuring the current and applying the current in the voltage/current measurement, when the measured current is less than a given standard current at a given time-point, after the given time-point, it is preferable to apply the current and to measure the current further until the measured current becomes equal to or more than the given standard current.

For example, in the case of -0.2 mA/cm² of the standard current, when the measured current is -0.3 mA/cm² at a given time-point, after the given time-point, it is allowed to apply the current and to measure the current further until the measured current becomes -0.1 mA/cm².

The given standard voltage or the standard current can be a voltage or a current measured in a healthy person.

When the measured value is a voltage, the measured value is compared with the standard voltage to determine their magnitude relation, and when the measured value is a current, the measured value is compared with the standard current to determine their magnitude relation.

In applying the voltage or the current, the voltage can be applied to set the electric potential of the epidermal surface at the distal end of the other probe with respect to the dermis near the distal end of the hollow needle in the range of -3 V or more and less than 0 V, preferably -2 V or more and less than 0 V. The applied current can be -1 to 0 mA/cm², preferably -0.5 to 0 mA/cm². The application time can be 0 to 180 minutes, preferably 0 to 120 minutes.

According to such embodiments, after detecting the deterioration of the barrier function in the epidermal electric potential measurement, it is possible to further measure the epidermal electric potential with improving the barrier function of the skin by applying the voltage or the current to the skin. Moreover, according to these embodiments, from other viewpoints, it is possible to improve the barrier function of the skin with monitoring the epidermal electric potential in the epidermal electric potential measurement.

In some of the embodiments, the preferable embodiments include also the following.

In measuring the voltage and applying the voltage in the voltage/current measurement, when the measured voltage is less than a given standard voltage at a given time-point, it is preferable to stop measuring the voltage.

For example, in the case of -10 mV of the standard voltage, when the measured voltage is -15 mV at a given time-point, it is allowed to stop measuring the voltage.

In measuring the current and applying the current in the voltage/current measurement, when the measured current is more than a given standard current at a given time-point, it is preferable to stop measuring the voltage.

For example, in the case of -0.2 mA/cm² of the standard voltage, when the measured current is -0.1 mA/cm² at a given time-point, it is allowed to stop measuring the voltage.

According to these embodiments, it is possible to measure the epidermal electric potential in the minimally requiring time without causing unnecessary damage or influence to the skin.

### (Cosmetic method)

A cosmetic method in some of the embodiments employs the epidermal electric potential measuring device in some of the embodiments and comprises performing probe disposition which includes disposing the probe to make its distal end reach the dermis and disposing the other probe to make its distal end positioned on the skin surface, and performing voltage/current application which includes applying a voltage or a current between the first electrode and the second electrode.

The cosmetic method in some of the embodiments does not preferably correspond to a medical therapeutic method.

A subject of the cosmetic method of the present embodiment can be human or non-human.

### ((Probe Disposition))

The probe disposition in the cosmetic method in some of the embodiments can be the same as the probe disposition in the epidermal electric potential measuring method in some of the embodiments described above.

### ((Voltage/current Application))

In the voltage/current application in the cosmetic method in some of the embodiments, the voltage can be applied to set the potential of the epidermal surface at the distal end of the other probe with respect to the dermis near the distal end of the hollow needle in the range of -3 V or more and less than 0 V, preferably -2 V or more and less than 0 V. From the point of obtaining a healthy skin with the functional barrier, the negative (minus) is preferable for the electric potential of the other probe to the electric potential of the probe, and also the larger is preferable for the absolute value of the negative (minus) value.

The applied current in the voltage/current application can be -1 to 0 mA/cm², preferably -0.5 to 0 mA/cm². From the point of obtaining a healthy skin with the functional barrier, the larger is preferable for the current flow from the probe to the other probe and, for example, the less is preferable for the absolute value of the negative (minus) value when the current is negative (minus).

The application time can be 0 to 180 minutes, preferably 0 to 120 minutes.

According to the cosmetic method in some of the embodiments, it is possible to obtain the effect of improving the barrier function of the skin.

Although the embodiments of the probe, the epidermal electric potential measuring device, the epidermal electric potential measuring method, and the cosmetic method of the present disclosure have been exemplified and described with reference to the figures, the above-described embodiments can be modified as appropriate, and the probe, the epidermal electric potential measuring device, the epidermal electric potential measuring method, and the cosmetic method of the present disclosure are not limited to the above- exemplified embodiments.

### EXAMPLES

The present disclosure will be described in more detail with reference to Examples below, but the present disclosure is not limited to the following Examples at all.

### A. Materials

### A-1. Reagents

- Agarose (manufactured by DOJINDO LABORATORIES)
- NaCl (manufactured by Wako Pure Chemical Industries, Ltd.)
- KCl (manufactured by Wako Pure Chemical Industries, Ltd.)
- CaCl₂·2H₂O (manufactured by Wako Pure Chemical Industries, Ltd.)
- HCl (manufactured by Wako Pure Chemical Industries, Ltd.)
- γ-butyrolactone (manufactured by Sigma Aldrich, Inc.)
- Acetone (manufactured by Wako Pure Chemical Industries, Ltd.)

### A-2. Animals

- Hairless mouse HR-1 (Hoshino Laboratory Animals, Inc.)
- Human subject(36 years old, healthy man)

### A-3. Supplies

- Nanopass Needle II 34 G (outer diameter 0.18 mm, length 4 mm, manufactured by TERUMO Corp.)
- Silicone rubber tube (inner diameter 3 mm, outer diameter 5 mm, manufactured by AS ONE Corp.)
- Rubber tube (inner diameter 7.9 mm, outer diameter 11.2 mm, manufactured by SAINT-GOBAIN)
- Biopsy punch (manufactured by Kai Medical Inc.)
- Glass tube (inner diameter 2 mm, outer diameter 3 mm, manufactured by IWAKI Co., Ltd.)
- Pt wire (diameter 0.4 mm, manufactured by Tanaka Kikinzoku Kogyo Co., Ltd.)
- Ag wire (diameter 0.5 mm, manufactured by Tanaka Kikinzoku Kogyo Co., Ltd.)
- Ag/AgCl electrode (manufactured by BAS Inc.)
- Parafilm (manufactured by AS ONE Corp.)
- Silicone rubber sheet (manufactured by AS ONE Corp.)
- Skin collected from pigs (manufactured by Funakoshi Co., Ltd.)
- Cellophane tape (manufactured by Nichiban Co., Ltd.)
- Cotton (manufactured by Nichiban Co., Ltd., manufactured by White Cross Corporation)

### A-4. Apparatus

- Desktop type surface treatment apparatus (Model SSP 17-110, SenLights Co.): for UV ozone treatment
- Electrochemical Analyzer (ALS 760C, manufactured by BAS Inc.): Voltmeter
- Water transpiration measuring instrument (H4300, manufactured by NIKKISO THERM Co., Ltd.)

### B. Fabrication of the epidermal electric potential measuring device

### B-1. Preparation of the salt bridge needle

0.02 g of agarose powder solution was added to 1 mL of Ringer's solution (prepared by dissolving 4.3 g NaCl, 0.15 g KCl, 0.165 g CaCl₂·2H₂O in 500 mL distilled water) and heated to dissolve the powder to prepare a 2 wt% agarose solution.

A nanopass needle was treated by ultraviolet ozone (condition: intensity 15 mW·cm² for 5 minutes exposure) to make the surface hydrophilic (contact angle of the outer surface 5°).

The surface-treated nanopass needle was immersed in distilled water warmed to about 50 °C to warm the entire needle.

The 2 wt% agarose solution was injected into the warmed nanopass needle from its terminal end (cartridge side) with a syringe and cooled to room temperature to fill the inside of the needle with the agarose gel.

A hole was made with biopsy punch (diameter 5 mm) on the lateral side of the cartridge of the nanopass needle to insert a salt bridge rubber tube (described later).

The obtained hollow needle for the probe was stored in the Ringer's solution until immediately before use.

When the hollow needle for the probe was used, a silicone rubber ring (inner diameter 3 mm, outer diameter 6 mm, thickness 2.5-3 mm, manufactured by AS ONE Corp.) was mounted as a spacer at the distal end of the needle.

### B-2. Preparation of the salt bridge rubber tube

0.02 g of agarose powder solution was added to 1 mL of Ringer's solution (prepared by dissolving 4.3 g NaCl, 0.15 g KCl, 0.165 g CaCl₂·2H₂O in 500 mL distilled water) and heated to dissolve the powder to prepare a 2 wt% agarose solution.

The 2 wt% agarose solution was injected into a silicone rubber tube (inner diameter 3 mm, outer diameter 5 mm), and cooled to room temperature to fill the inside of the tube with the agarose gel.

### B-3. Preparation of the Ag/AgCl electrode

A glass tube was cut to a length of 20 mm.

Pt wire (diameter 0.4 mm, length 10 mm) was inserted into the glass tube from its one end, heated at the site with gas burner to melt the glass, and then sealed in the glass tube.

Saturated KCl aqueous solution was injected from the opposite side of the sealed side and filled in the tube.

KCl powder and AgCl powder were added into the glass tube to be precipitated at the distal end.

Ag wire (working electrode), Pt wire (counter electrode) and Ag/AgCl electrode (reference electrode) were immersed in 1 mM HCl aqueous solution. A constant electric potential of 0.6 V was applied to the Ag wire for 300 seconds to precipitate AgCl electrolytically on the surface of the wire so that the Ag/AgCl wire was prepared.

The Ag/AgCl wire was inserted into the glass tube in which KCl and AgCl were precipitated, and was sealed in the tube with parafilm.

The obtained Ag/AgCl electrode was immersed in saturated KCl aqueous solution until immediately before use.

### B-4. Fabrication of the epidermal potential measuring device (integrated type)

The integrated type of the epidermal electric potential measuring device having the configuration illustrated in FIG. 2 was fabricated.

The cartridge part was removed from the nanopass needle and only the needle part was isolated.

The isolated needle part was inserted into the silicone rubber tube from its one end, and 2 wt% agarose solution prepared with Ringer's solution was injected from the other end of the tube to be filled in. This was cooled to room temperature and the agarose gel was prepared in the tube.

The above-described Ag/AgCl electrode was inserted into the rubber tube from its other end (the same end from which the agarose solution was injected).

A silicone rubber ring (inner diameter 1.5 mm, outer diameter 4 mm, thickness 2 mm, manufactured by AS ONE Corp.) was mounted as a spacer at the distal end of the nanopass needle.

The needle part-agarose gel-Ag/AgCl electrode construction and the separately prepared Ag/AgCl electrode were inserted into a larger-sized rubber tube (inner diameter 7.9 mm, outer diameter 11.2 mm).

Then, the 2 wt% agarose solution prepared with Ringer's solution was poured into space at the distal end of the separately prepared Ag/AgCl electrode and cooled and solidified.

### B-5. Fabrication of the epidermal electric potential measuring device (separate type)

Following the method of B-4, separate type of the epidermal electric potential measuring device having the configuration illustrated in FIG. 1 was also fabricated.

### C. Epidermal electric potential measurement

### C-1. Epidermal electric potential measurement of hairless mouse

The epidermal electric potential measuring method illustrated in FIG. 3 was applied.

FIGS. 5A and 5B illustrate an outline of the experiment when the epidermal electric potential of a hairless mouse is measured by one example of epidermal electric potential measuring method according to some of the embodiments of the disclosure. FIG. 5A is a diagram of the principle of the experiment, and FIG. 5B is a photograph of the appearance during the experiment.

The room temperature in the animal laboratory was set at 23 °C and the humidity was set at 25 %.

The hairless mouse was injected with an anesthetic (γ-butyrolactone, diluted 30-fold in PBS solution) and was subject to the following procedure under anesthesia.

The moisture transpiration amount was measured at each of the skin on the left and right flanks.

Using the setup of FIGS. 5A and 5B, the epidermal electric potential was measured with placing the distal end of the salt bridge tube (the other probe) in contact with the skin surface of both the left and the right flank and with inserting the salt bridge needle (the probe) into the dermis of the back.

For comparison with the conventional method, in the setup illustrated in FIGS. 5A and 5B, the epidermal electric potential was also measured with placing the salt bridge tube in contact with the skin surface of both the left and the right flank and with placing the distal end of the salt bridge, from which the salt bridge needle was removed, in contact with a wound (about 3 mm square) formed on the back of the mouse.

At the measuring site on the left flank, the stratum corneum was removed by repeating the operation of sticking a cellophane tape (1 cm square) and then peeling it off 2 to 3 times.

At the measuring site of the right flank, the stratum corneum was degreased by applying the cotton impregnated with acetone (1 cm square) for 5 minutes.

The amount of moisture transpiration and the epidermal electric potential were measured at the stratum corneum removed site and the degreased site according to the above-described method.

FIGS. 6A and 6B illustrate the results of measuring the epidermal electric potentials of the hairless mice by one example of epidermal electric potential measuring method according to some of the embodiments of the disclosure. FIG. 6A is a graph of the results when stratum corneum removing treatment was given, and FIG. 6B is a graph of the results when degreasing treatment was given. In FIG. 6A and FIG. 6B, vertical lines indicate error bars (mean ± standard deviation) of three measurements.

FIGS. 6A and 6B illustrate the epidermal electric potential measured before and after the stratum corneum removing treatment and the degreasing treatment. The averages of the measured values for 60 seconds are indicated as bar graphs.

Completion of the stratum corneum removing treatment and the degreasing treatment was determined by detecting the increase of the amount of water transpiration from the skin by 50% or more with the measuring instrument.

Each treatment reduced the epidermal electric potential (white bar in FIGS. 6A and 6B), which was the typical change similar to that of values in the past literature. In addition, the obtained values are almost the same as values (not shown) obtained by the conventional measuring method (measuring method in which the salt bridge rubber tube is placed in contact with the wound formed on the back of a mouse as the internal electrode). Furthermore, the variances of three measurements (measured values with 3 mice independently) were small.

From the above, reproducibility and quantitativeness were confirmed in the epidermal electric potential measuring method which employs the salt bridge needle.

### C-2. Epidermal electric potential measurement of porcine skin (detailed examination)

The epidermal electric potential measuring method illustrated in FIG. 3 was applied.

FIGS. 7A and 7B illustrate an outline and results of investigating the preferable distance between the distal end of the probe according to some of the embodiments of the disclosure and the distal end of the other probe according to some of the embodiments of the disclosure by using porcine skin. FIG. 7A is a diagram for the outline of the experiment, and FIG. 7B is a graph presenting the results of the experiment.

The porcine skin was soaked in Ringer's solution to less than half its thickness.

Two containers were prepared to be filled with saturated KCl aqueous solution and one Ag/AgCl electrode was immersed in each container.

One end of the salt bridge rubber tube was immersed in one of the prepared containers and the other end was placed in contact with the porcine skin surface to be used as a surface electrode (preparation of the other probe).

One end of the other salt bridge rubber tube was immersed in the other prepared container and the other end was inserted into the lateral side of the cartridge of the salt bridge needle (preparation of the probe).

Two Ag/AgCl electrodes were connected to a voltmeter and the measurement of the electric potential difference between these two electrodes was started.

Two seconds after starting the measurement, the salt bridge needle was inserted into the porcine skin as the internal electrode and the epidermal electric potential was measured.

In measuring the electric potential difference inside the dermis, the epidermis was removed by the biopsy punch (diameter 8 mm) at any one site on the porcine skin surface, and the distal end of the salt bridge rubber tube (the distal end of the other probe) was placed in contact with the site, while the salt bridge needle was inserted into the skin until its distal end (the distal end of the probe) reached the dermis.

The epidermal electric potential was determined as the value subtracted by the electric potential difference (about 5 mV) between the two Ag/AgCl electrodes themselves from the measured electric potential.

As illustrated in FIG. 7B, the electric potential difference was measured between the salt bridge rubber tube disposed on the skin surface (surface electrode) and the salt bridge needle electrode inserted into the dermal layer.

As indicated in FIG. 7B, since the inside of the dermal layer is equipotential, the measured electric potential difference reflects the epidermal electric potential right under the surface electrode. Immediately after inserting the salt bridge needle, stable electric potential of about -15 mV was able to be measured (solid line in FIG. 7B). In addition, even if the distance between the salt bridge needle and the surface electrode was shortened from 20 mm to 10 mm, further to 2 mm, the skin electric potential was measured as almost the same value (long broken line (for 10 mm) and short broken line (for 2 mm) in FIG. 7B).

This demonstrated that the insertion of the salt bridge needle near the surface electrode did not affect the electric potential measuring.

This suggests that it is possible to integrate the surface electrode and the internal needle electrode into one probe.

### C-3. Epidermal electric potential measurement of porcine skin (detailed examination)

The epidermal electric potential measuring method illustrated in FIG. 3 was applied. However, at this time, the epidermis removing treatment was given to the surface of the skin on which the distal end of the second electrode probe would be located.

FIGS. 8A, 8B, and 8C illustrate an outline and results of investigating the preferable length of the hollow needle in the probe according to some of the embodiments of the disclosure and the preferable treatment for the outer surface of the hollow needle by using porcine skin subjected to epidermis removing treatment. FIG. 8A is a diagram for the outline of the experiment, and FIGS. 8B and 8C are graphs presenting the results of the experiment. In FIG. 8C, vertical lines indicate error bars (mean±standard deviation) of three measurements.

The stability was investigated for the electric potential after inserting the salt bridge needle into the skin. Using the setup of FIG. 8A, the epidermal electric potential was measured with placing the distal end of the salt bridge rubber tube in contact with the epidermis removed site and with inserting the other salt bridge needle into the skin. At this time, since both electrodes are installed in the dermal layer of the skin, no potential difference is ideally generated.

As illustrated in FIGS. 8B and 8C, the salt bridge needle was inserted two seconds after starting the measurement. The lines in the graph fluctuated before the insertion as the electric potential was not able to be measured, but the stable electric potential of about 0 mV (no potential difference) was measured immediately after the insertion (solid line in FIG. 8B). On the other hand, the measured electric potential difference was greatly deviated from 0 mV in the absence of the spacer at the distal end (short broken line in FIG. 8B) and the values greatly varied from electrode to electrode. This is probably because the distal end of the needle reached the hydrophobic subcutaneous fat layer to affect the measured electric potential. Moreover, even if the distal end of the needle was clamped to the dermal layer by mounting the spacer to the needle, rapid transition to 0 mV and stabilization of the electric potential were not observed without hydrophilizing treatment by UV ozone (long dashed line in FIG. 8B). This seems that the hydrophobicity of the nanopass needle surface prevented the rapid contact between the interstitial fluid in the dermal layer and the agarose gel inside the needle.

FIG. 8C demonstrates the electric potential drift amount from 10 seconds to 60 seconds after starting the measurement. The electric potential drift was small for the salt bridge needle mounted with the spacer and hydrophilized (white bar in FIG. 8C). From the above results, the salt bridge needle mounted with the spacer and hydrophilized made it possible to measure the electric potential quickly and stably by being inserted into an appropriate depth.

### C-4. Epidermal electric potential measurement of porcine skin

The epidermal electric potential measuring method illustrated in FIG. 4 was applied.

FIGS. 9A and 9B illustrate an outline and results of measuring the epidermal electric potentials of porcine skins by another example of epidermal electric potential measuring method according to some of the embodiments of the present disclosure. FIG. 9A is a photograph of the epidermal potential measuring device, FIG. 9B presents a photograph and a graph for the outline and the results of the experiment, respectively, when stratum corneum removing treatment was given, and another photograph and another graph for the outline and the results of the experiment, respectively, when degreasing treatment was given. In FIG. 9B, A and C represent the untreated sites, and B represents the treated site.

The surface electrode and internal needle integrated-probe was prepared (FIG. 9A). The distance between the distal end of the injection needle and the distal end of the salt bridge tube in the direction perpendicular to the axial direction was set to 2 mm which is the shortest distance between the electrodes examined in FIG. 7B. In addition, the distance between the distal end of the injection needle and the distal end of the salt bridge tube in the axial direction was set to 1 mm. The stratum corneum removed site or the degreased site was formed in the local area of the porcine skin (1 cm square), and the difference of the epidermal electric potential to the surrounding region was measured with the probe electrode.

FIG. 9B indicates the epidermal electric potential of the porcine skin measured with the integrated-probe electrode. The probe was continuously placed in contact with A (normal), B (treated) and C (normal) sites represented in the photo for 100 seconds each, and the epidermal electric potential was measured at each time. In FIG. 9B, bar graphs present the averages of the epidermal electric potentials for 50 seconds out of the measurement time of 100 seconds. The epidermal electric potential was decreased at the treated site, and that is the same result as obtained with the hairless mice. In addition, the measured values were almost equal to the ones by the conventional method (measuring method in which a part of the epidermis is removed from the porcine skin and the salt bridge rubber tube is placed in contact with the site as the internal electrode), and quantitativeness in the repeated measurements was also able to be demonstrated.

### C-5. Epidermal electric potential measurement for human

The epidermal electric potential measuring method illustrated in FIG. 3 was applied. However, at this time, the degreasing treatment with acetone was given to the skin surface on which the distal end of the other probe would be located.

FIGS. 10A and 10B illustrate an outline of measuring the epidermal electric potential on the human forearm by another example of epidermal electric potential measuring method according to some of the embodiments of the present disclosure. FIG. 10A is a diagram for the outline of the experiment, and FIG. 10B is a photograph of the appearance during the experiment.

The temperature and the humidity in the room were measured (temperature: 28.4 °C, humidity: 40.4 %).

The two electrodes (the rubber tube for the surface electrode and the salt bridge needle for the internal electrode) were located on the upper arm indicated in the photograph of FIG. 10B, and the epidermal electric potential was measured. The distance between the electrodes was set to 4 cm. The insertion depth of the needle electrode was set to 1.5 mm. There was no repeat of putting in and out the needle in its insertion. The distal end of the needle was confirmed to reach the dermis when the electric potential difference, in inserting two needles by the similar procedure, was measured to be equivalent to the electric potential difference between the two Ag/AgCl electrodes themselves.

Once the electrode was taken off, and then cotton (1 cm square) impregnated with acetone was made contact with the site, where the surface electrode would be located, for 5 minutes to degrease the stratum corneum.

The arrangement of the probes and the like was restored and the epidermal electric potential was measured after the degreasing treatment.

At this time, by putting a 2 cm square collagen film (CLF-01, manufactured by Takashi Kenkyu Co., Ltd., impregnated with Ringer's solution and used) between the surface electrode and the skin, the area of the surface electrode was expanded to cover the whole degreased site (see FIG. 10A).

Thereafter, a constant voltage of -0.5 V to the internal electrode was applied to the surface electrode for 1 hour. For the comparative examples, the collagen film was merely stuck to the degreased site and no voltage was applied.

The collagen film was peeled off, the arrangement of the probes and the like was restored and the epidermal electric potential was measured after the electric stimulation.

The epidermal electric potential was determined as the value subtracted by the electric potential difference between the two Ag/AgCl electrodes themselves from the measured electric potential.

FIGS. 11A and 11B illustrate results of measuring the epidermal electric potential on the human forearm by another example of epidermal electric potential measuring method according to some of the embodiments of the present disclosure. FIG. 11A is a graph for the results when a voltage is applied for 1 hour after degreasing treatment, and FIG. 11B is a graph for the results when no voltage is applied to be left alone for 1 hour after degreasing treatment.

FIGS. 11A and 11B illustrate the epidermal electric potential measured before and after the degreasing treatment with acetone and the electric stimulation. The averages of the measured values for 60 seconds are indicated in bar graphs. After the degreasing treatment, the epidermal electric potential was decreased similarly as was seen in the case of the hairless mice. When a constant voltage of -0.5 V was applied to the surface electrode for 1 hour, the epidermal electric potential was increased (white bar in FIG. 11A). On the other hand, when the epidermis was left alone for 1 hour without the stimulation, the epidermal electric potential was decreased (hatched bar in FIG. 11B). From these results, it was demonstrated that the electrical stimulation according to the present disclosure is effective for the increase in the epidermal electric potential, that means, the recovery.

### INDUSTRIAL APPLICABILITY

According to the present disclosure, the minimally invasive epidermal electric potential measurement can be provided by inserting a painless needle at an appropriate depth. The epidermal electric potential measuring device and the epidermal electric potential measuring method according to the present disclosure have a possibility of greatly contributing to the treatment and the diagnosis for the barrier dysfunction of the skin.

## Claims

1. A probe comprising a hollow needle having an outer diameter of 0.003 to 0.4 mm and a length of 0.2 to 40 mm, wherein a distal end and a terminal end of the hollow needle are bridged with a salt bridge.

2. The probe according to claim 1, wherein a contact angle of water to the outer surface of the hollow needle is 1 to 175 °.

3. The probe according to claim 1 or 2, wherein the outer surface of the hollow needle is treated with ultraviolet ozone.

4. The probe according to any one of claims 1 to 3, wherein the salt bridge comprises a hydrogel.

5. The probe according to any one of claims 1 to 4, wherein the hydrogel comprises a hydrogel material and a physiological salt solution.

6. An epidermal electric potential measuring device comprising: the probe according to any one of claims 1 to 5; a first electrode bridged to the terminal end of the hollow needle with the salt bridge; and a second electrode connected to the first electrode by an electron conductive material; and another probe bridged to the second electrode with the salt bridge.

7. The epidermal electric potential measuring device according to claim 6, wherein a distance between the distal end of the probe and the distal end of the other probe in a direction perpendicular to an axial direction of the hollow needle is 1 to 50 mm.

8. The epidermal electric potential measuring device according to claim 6 or 7, wherein a spacer is provided between the distal end and the terminal end of the hollow needle.

9. An epidermal electric potential measuring method using the epidermal electric potential measuring device according to any one of claims 6 to 8, the method comprising:
performing probe disposition including disposing the probe to make its distal end reach the dermis and disposing the another probe to make its distal end positioned on the skin surface; and
performing voltage/current measurement including measuring a voltage by applying a current or measuring a current by applying a voltage between the first electrode and the second electrode.

10. A cosmetic method using the epidermal electric potential measuring device according to any one of claims 6 to 8, and comprising:
performing probe disposition including disposing the probe to make its distal end reach the dermis and disposing the other probe to make its distal end positioned on the skin surface; and
performing voltage/current application including applying a voltage or a current between the first electrode and the second electrode.
